# EUROPEAN PATENT SPECIFICATION

(11) **EP 2 974 749 B1**
(45) Date of publication and mention of the grant of the patent: **16.11.2016**
(21) Application number: 14763511.4
(22) Date of filing: 26.02.2014
(51) Int. Cl.: A61L 9/22, B05B 5/057, H01T 23/00, B05B 5/025

(54) **ACTIVE INGREDIENT GENERATING DEVICE**
WIRKSTOFFERZEUGUNGSVORRICHTUNG
DISPOSITIF DE GÉNÉRATION D'INGRÉDIENT ACTIF

(30) Priority: 11.03.2013 JP 2013048090
(43) Date of publication of application: 20.01.2016
(73) Proprietor: Panasonic Intellectual Property Management Co., Ltd., Osaka-shi, Osaka 540-6207 (JP)
(72) Inventor: MACHI, Masaharu, Chuo-ku, Osaka-shi, Osaka 540-6207 (JP); ISHIGAMI, Youhei, Chuo-ku, Osaka-shi, Osaka 540-6207 (JP); MISHIMA, Yukiko, Chuo-ku, Osaka-shi, Osaka 540-6207 (JP); KOMURA, Yasuhiro, Chuo-ku, Osaka-shi, Osaka 540-6207 (JP); SUMIMOTO, Ayaka, Chuo-ku, Osaka-shi, Osaka 540-6207 (JP)
(74) Representative: Grünecker Patent- und Rechtsanwälte PartG mbB
(86) International application number: PCT/JP2014/001031
(87) International publication number: WO 2014/141604

(56) References cited:
- JP-A- S60 241 445
- JP-A- 2000 107 634
- JP-A- 2000 107 634
- JP-A- 2002 143 634
- JP-A- 2002 143 634
- JP-A- 2008 104 982
- US-A- 3 337 784

## Description

### Technical Field

The present invention relates to a device which generates active ingredients such as radicals having effects such as deodorization and sterilization.

### Background Art

Radicals are useful for various purposes such as deodorization and sterilization. Patent Document 1 discloses an electrostatic atomizer which generates fine water particles containing radicals (charged particulate water). The electrostatic atomizer of Patent Document 1 includes an atomizing electrode, a counter electrode and a liquid feeder. The liquid feeder feeds liquid to a distal end of the atomizing electrode. The electrostatic atomizer causes a discharge between the atomizing electrode and the counter electrode. As the result of the discharge, there is atomization of the liquid fed to the atomizing electrode. The atomized liquid becomes charged particulate water containing radicals.

When a discharge is caused under application of a high voltage to an electrode, not only radicals but also ozone is generated. Ozone has useless characteristics such as nasty smell. Therefore, there is a problem about a limited yield of radicals since an amount of ozone increases even if it is desired to increase a yield of radicals.

JP 2002-143634 A discloses the preamble of claim 1 and relates to a high-voltage device provided with an air treating function using a plasma deodorizing unit.

Patent Document 1: JP 2007-313460 A

### Summary of Invention

An object of the present invention is to provide an active ingredient generator which reduces a yield of ozone as compared to a yield of radicals.

An active ingredient generator according to the present invention is defined in claim 1. Further advantageous embodiments are defined in the dependent claims.

The active ingredient generator according to the present invention may reduce an emission amount of ozone.

Other objects, features and advantages of the present invention will become more apparent from the following detailed description and accompanying drawings.

### Brief Description of Drawings

Fig. 1 is a schematic view of a radical generator exemplified as the active ingredient generator according to the first embodiment.
Fig. 2 is a schematic view of an electrostatic atomizer exemplified as the active ingredient generator according to the second embodiment.
Fig. 3 is a graph schematically showing radical amounts under the presence and absence of a glow discharge.
Fig. 4 is a graph schematically showing ozone amounts under the presence and absence of a glow discharge.
Fig. 5 is a schematic view of an electrostatic atomizer exemplified as the active ingredient generator according to the third embodiment.
Fig. 6 is a schematic view of an electrostatic atomizer exemplified as the active ingredient generator according to the fourth embodiment.
Fig. 7 is a schematic view of an electrostatic atomizer exemplified as the active ingredient generator according to the fifth embodiment.
Fig. 8 is a schematic view of an electrostatic atomizer exemplified as the active ingredient generator according to the sixth embodiment.
Fig. 9 is a partial enlarged view of a first discharger around a center line shown in Fig. 8.
Fig. 10 is a schematic graph showing a relationship between an angle θ illustrated in Fig. 9 and an ion flow velocity.
Fig. 11 is a schematic graph showing a relationship between a discharge distance and an ion flow velocity.
Fig. 12 is a schematic graph showing a relationship between an ion flow velocity and a thickness of a first counter electrode of the electrostatic atomizer illustrated in Fig. 8.
Fig. 13 is a schematic graph showing a relationship between electric field intensity and a yield of active ingredients.
Fig. 14 is a graph showing an exemplary control of voltage application to a first discharger and a second discharger.
Fig. 15 is a schematic graph showing a relationship between a yield of ozone and an opening size of a cylindrical portion of the electrostatic atomizer illustrated in Fig. 8.
Fig. 16 is a schematic view of an electrostatic atomizer exemplified as the active ingredient generator according to the seventh embodiment.
Fig. 17 is a schematic view of an electrostatic atomizer exemplified as the active ingredient generator according to the eighth embodiment.

### Description of Embodiments

Exemplary active ingredient generators are described below. Directional terms such as "up", "down", "left" and "right" are used just for clarifying the following description. Therefore, these terms do not limit principles of the active ingredient generators.

### <First Embodiment>

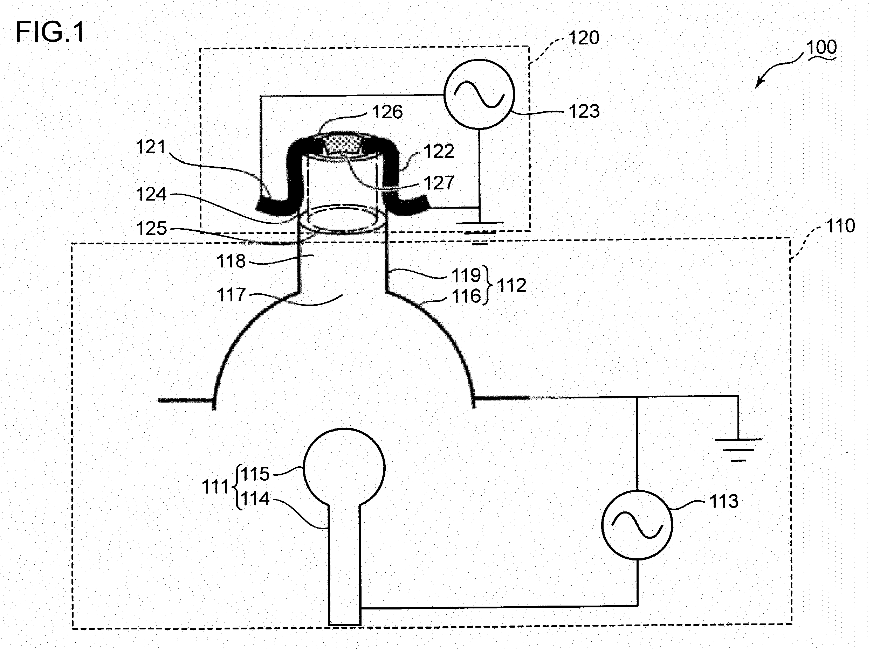

Fig. 1 is a schematic view of a radical generator 100 exemplified as the active ingredient generator according to the first embodiment. The radical generator 100 is described with reference to Fig. 1.

The radical generator 100 includes a first discharger 110 and a second discharger 120. The first discharger 110 causes a first discharge having first energy. The second discharger 120 causes a second discharge having second energy larger than the first energy.

The first discharge generates radicals and ozone. Fluid including the radicals and ozone is delivered to the second discharger 120 by ion flow or other fluid control techniques. Accordingly, the fluid delivered from the first discharger 110 is exposed to the second discharge. Since the second energy of the second discharge is larger than the first energy of the first discharge, there is a decrease in an amount of ozone.

The first discharger 110 includes a first discharge electrode 111, a first counter electrode 112 and a first power supply 113. The first discharge electrode 111 includes a rod-shaped body 114 and a spherical head 115. The body 114 is electrically connected to the first power supply 113. The first power supply 113 applies an AC voltage to the first discharge electrode 111. Accordingly, a corona discharge occurs at the head 115 connected to the upper end of the body 114. For example, the first power supply 113 may include a circuit which uses piezoelectric elements. In the present embodiment, the first power supply 113 applies an AC voltage. Alternatively, the first discharge may be caused by application of a DC voltage.

The first counter electrode 112 faces the first discharge electrode 111. The first counter electrode 112 includes a curved portion 116 which is curved upward in a dome shape. The curved portion 116 defines an opening 117 which faces the first discharge electrode 111. The first counter electrode 112 further includes a cylinder 119 which defines a passage 118 in communication with the opening 117.

The first counter electrode 112 situated above the first discharge electrode 111 is grounded. Since a potential difference occurs between the first discharge electrode 111 and the first counter electrode 112, electric energy which the first power supply 113 supplies to the first discharge electrode 111 is discharged as the first energy from the first discharge electrode 111 to the first counter electrode 112. Therefore, a space between the first discharge electrode 111 and the first counter electrode 112 is used as a discharge space in which the first discharge is caused.

Since the first power supply 113 applies an AC voltage to the first discharge electrode 111 as described above, radicals generated under the first discharge reciprocate between the first discharge electrode 111 and the first counter electrode 112. Accordingly, there is an increase in plasma density in the discharge space between the first discharge electrode 111 and the first counter electrode 112. Therefore, radicals are efficiently generated in the discharge space.

The radicals and ozone generated in the discharge space flow into the passage 118 through the opening 117. The radicals and ozone then flow into the second discharger 120.

The first discharger 110 may be stored in a housing (not shown) or a container (not shown) which defines a closed space. The cylinder 119 may be used as a passage for emitting air, which contains radicals and ozone, from the housing or the container.

The second discharger 120 includes a second discharge electrode 121, a second counter electrode 122, a second power supply 123 and a supporting cylinder 124. The supporting cylinder 124 includes a lower edge 125, which is connected to the upper edge of the cylinder 119, and an upper edge 126 opposite to the lower edge 125. The supporting cylinder 124 defines the passage 118 in cooperation with the cylinder 119. The upper edge 126 defines an emission port 127 which allows emission of radicals and ozone generated in the discharge space. Since fluid flowing from the discharge space passes through the emission port 127 and a space near the emission port 127, the emission port 127 and the space near the emission port 127 are exemplified as the passage space.

The second discharge electrode 121 and the second counter electrode 122 are attached to the upper edge 126. The second discharge electrode 121 and the second counter electrode 122 may be formed from a conductive wire. Fluid emitted from the emission port 127 passes between the second discharge electrode 121 and the second counter electrode 122 which faces the second discharge electrode 121.

The second discharge electrode 121 is electrically connected to the second power supply 123. The second counter electrode 122 is grounded. The second power supply 123 applies an AC voltage to the second discharge electrode 121 to generate a glow discharge between the second discharge electrode 121 and the second counter electrode 122. Therefore, the fluid emitted from the emission port 127 is exposed to the glow discharge. For example, the second power supply 123 may include a circuit which uses piezoelectric elements.

In the present embodiment, a glow discharge is used as the second discharge. Alternatively, other types of discharge (e.g. an arc discharge or a corona discharge) may be used as the second discharge. A glow discharge is superior to arc discharge in terms of a lower noise level and less electrode wear. The glow discharge is superior to a corona discharge in terms of an effective reduction in ozone.

The following chemical formula represents principles of an ozone reduction caused by a glow discharge.

[Chemical Formula 1] *O*₃+*e*→*O*₂+*O*

The glow discharge gives an electron to ozone generated between the first discharge electrode 111 and the first counter electrode 112. Accordingly, the ozone is split into an oxygen molecule and an oxygen atom.

The following chemical formula represents principles of an ozone reduction by the oxygen atom resultant from splitting ozone.

[Chemical Formula 2] *O*₃+*O*→2*O*₂

The oxygen atom splits ozone to produce two oxygen molecules.

A glow discharge gives an electron to an oxygen molecule in the air. Accordingly, the oxygen molecule is split into two oxygen atoms.

The following chemical formula represents generation of oxygen atoms resultant from splitting the oxygen molecule.

[Chemical Formula 3] *O*₂+*e*→2*O*

The following chemical formula represents principles of generation of radicals by the oxygen atom resultant from splitting the ozone or oxygen molecule.

[Chemical Formula 4] *O*+*H*₂*O*→2*OH*

The oxygen atom combines with a water molecule contained in the air passing through the emission port 127 to produce two OH radicals.

As described above, the glow discharge may reduce ozone and produce OH radicals.

### <Second Embodiment>

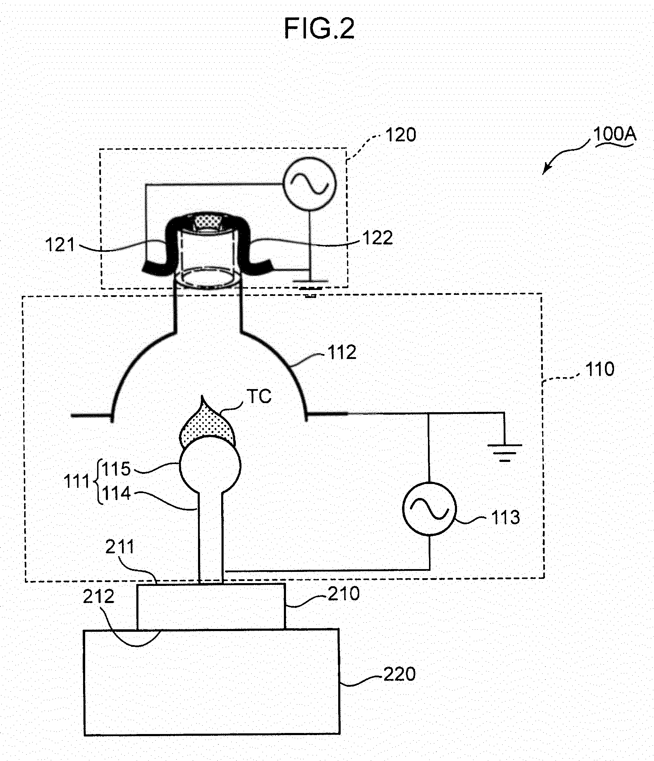

Fig. 2 is a schematic view of an electrostatic atomizer 100A exemplified as the active ingredient generator according to the second embodiment. The electrostatic atomizer 100A is described with reference to Fig. 2. The same components as the first embodiment are denoted by the same reference numerals. The description of the first embodiment is applicable to the components denoted by the same reference numerals.

Like the first embodiment, the electrostatic atomizer 100A includes the first and second dischargers 110, 120. The electrostatic atomizer 100A further includes a cooling module 210 and a heat radiating fin 220.

The cooling module 210 includes an upper surface 211, to which the body 114 is fixed, and a lower surface 212 opposite to the upper surface 211. The lower surface 212 is connected to the heat radiating fin 220. In the present embodiment, the cooling module 210 cools the first discharge electrode 111 using Peltier elements (not shown). The Peltier elements heat the lower surface 212 whereas the Peltier elements cool the upper surface 211.

A temperature of the first discharge electrode 111 goes down when the upper surface 211 is cooled. Accordingly, there is condensation on the first discharge electrode 111. When the first power supply 113 applies a voltage to the first discharge electrode 111, water adhering to the first discharge electrode 111 is gathered on the upper end of the head 115 by Coulomb's force to form a Taylor cone TC. A discharge then occurs via the Taylor cone TC between the first discharge electrode 111 and the first counter electrode 112. Accordingly, particulate water containing radicals is generated. In the present embodiment, the cooling module 210 is exemplified as the liquid feeder, the first feeder and/or the cooler.

The heat radiating fin 220 effectively emits heat of the lower surface 212. Accordingly, thermal failures are less likely to happen to the cooling module 210. In the present embodiment, the heat radiating fin 220 is exemplified as the first feeder and/or the radiator.

As described in the context of the first embodiment, the second discharge electrode 121 and the second counter electrode 122 cause a glow discharge. The particulate water containing radicals passes through a region in which the glow discharge occurs. Meanwhile, the glow discharge gives an electron to a surface of the particulate water. Accordingly, radicals are further generated. This means an increase in an amount of radicals contained in the particulate water.

The following chemical formula represents principles of generating radicals on the basis of resultant splitting from an electron fed to the particulate water.

[Chemical Formula 5] *e*+*H*₂*O*→*OH*+*H*

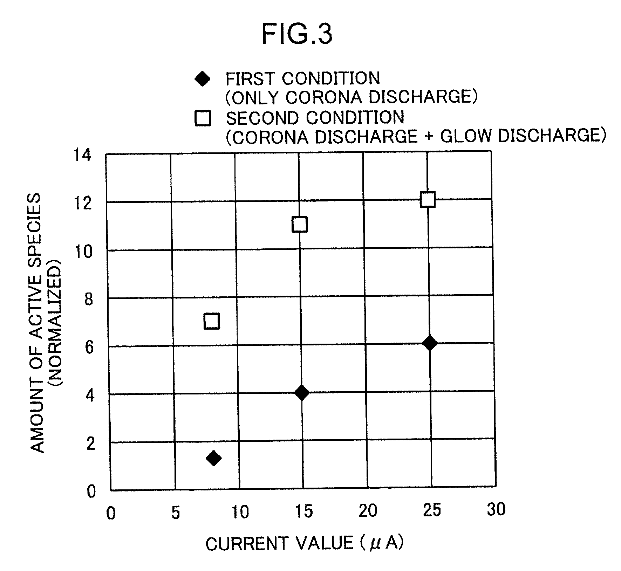

Fig. 3 is a graph schematically showing radical amounts under the presence and absence of a glow discharge. It is described with reference to Figs. 2 and 3 how the glow discharge affects a radical amount.

The present inventors used an electron spin resonance (ESR) to measure a radical amount. The radical amount may be measured by other measurement techniques. The principles of the present embodiment are not limited by measurement techniques of a radical amount.

The present inventors set a first condition, in which only a corona discharge happens to the first discharger 110, and a second condition, in which a corona discharge happens to the first discharger 110 whereas a glow discharge happens to the second discharger 120.

The vertical axis of the graph in Fig. 3 indicates an amount of active species by the value of "1", the active species being generated by a corona discharge, which is caused under a current value of 6 µA. As shown in Fig. 3, the radical amount is much larger under the second condition than the first condition. This means radical generation resultant from splitting a water molecule represented by "Chemical Formula 5" above. A part of the radicals generated by splitting a water molecule is contained in particulate water. Another part of the radicals generated by splitting a water molecule exists as isolated radicals. The radicals contained in the particulate water become particles which exist for a longer time than the isolated radicals do.

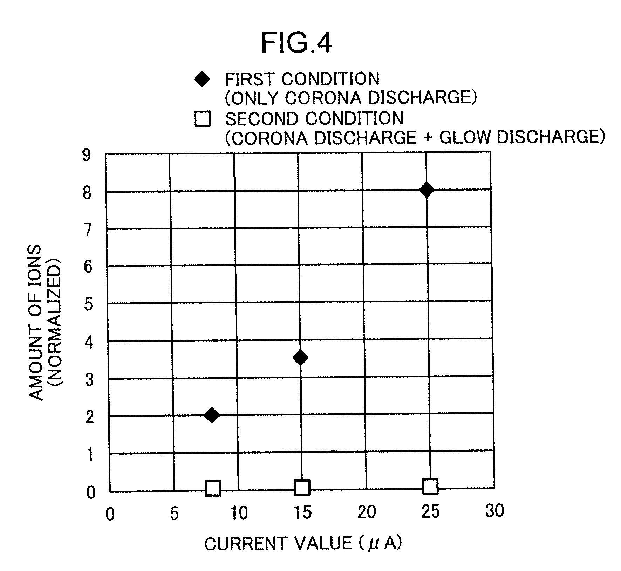

Fig. 4 is a graph schematically showing an ozone amount under the presence and absence of a glow discharge. It is described with reference to Figs. 2 and 4 how the glow discharge affects a radical amount.

The present inventors used a general ozone monitor to measure an ozone amount. The ozone amount may be measured by another measurement device. Principles of the present embodiment are not limited by measurement techniques of an ozone amount.

The present inventors set a first condition, in which only a corona discharge happens to the first discharger 110, and a second condition, in which a corona discharge happens to the first discharger 110 whereas a glow discharge happens to the second discharger 120.

The vertical axis of the graph in Fig. 4 indicates an ozone amount by the value of "1" when ozone is generated by a corona discharge caused under a current value of 6 µA. As shown in Fig. 4, the ozone amount is much smaller under the second condition than the first condition. This means that there are the phenomena represented by "Chemical Formula 1" above. The graph of Fig. 4 shows that a glow discharge effectively contributes to a reduction in ozone.

### <Third Embodiment>

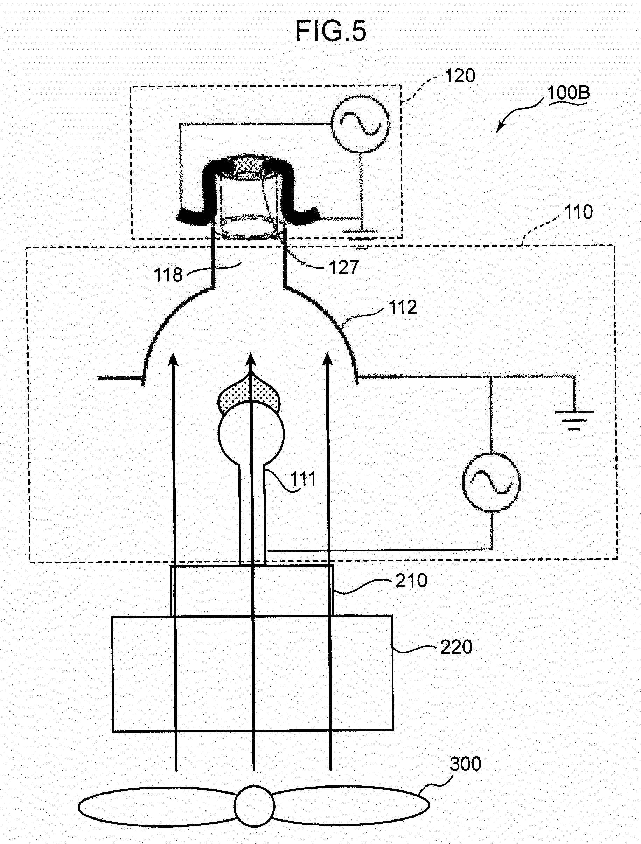

Fig. 5 is a schematic view of an electrostatic atomizer 100B exemplified as the active ingredient generator according to the third embodiment. The electrostatic atomizer 100B is described with reference to Fig. 5. The same components as the second embodiment are denoted by the same reference numerals. The description of the second embodiment is applicable to the components denoted by the same reference numerals as the second embodiment.

Like the second embodiment, the electrostatic atomizer 100B includes a first discharger 110, a second discharger 120, a cooling module 210 and a heat radiating fin 220. The electrostatic atomizer 100B further includes a blower 300.

The blower 300 situated below the heat radiating fin 220 delivers air toward the first counter electrode 112 to cause airflow into the passage 118 from the discharge space between the first discharge electrode 111 and the first counter electrode 112. Air containing radicals and ozone is forced to flow toward the emission port 127 by the airflow. Accordingly, an ozone amount is effectively reduced by a glow discharge. In the present embodiment, the blower 300 is exemplified as the blowing portion.

The heat radiating fin 220 is situated between the blower 300 and the first counter electrode 112. Therefore, the heat radiating fin 220 is exposed to the airflow caused by the blower 300. Accordingly, there is improved radiation efficiency from the heat radiating fin 220.

### <Fourth Embodiment>

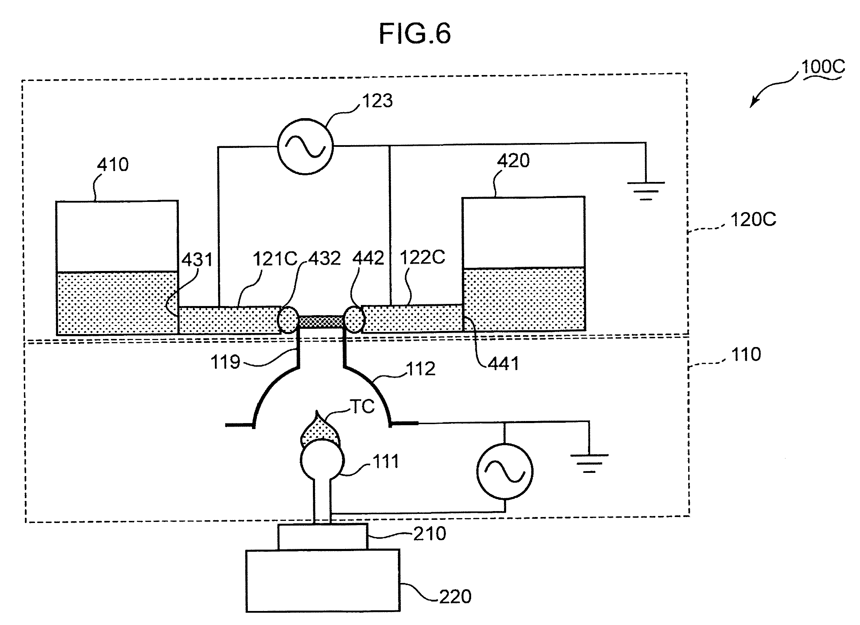

Fig. 6 is a schematic view of an electrostatic atomizer 100C exemplified as the active ingredient generator according to the fourth embodiment. The electrostatic atomizer 100C is described with reference to Fig. 6. The same components as the second embodiment are denoted by the same reference numerals. The description of the second embodiment is applicable to the components denoted by the same reference numerals as the second embodiment.

Like the second embodiment, the electrostatic atomizer 100C includes the first discharger 110, the cooling module 210 and the heat radiating fin 220. The electrostatic atomizer 100C further includes a second discharger 120C. Unlike the second embodiment, the electrostatic atomizer 100C uses not only the first discharger 110 but also the second discharger 120C to generate particulate water containing radicals. The particulate water containing radicals may be generated by the second discharger 120C only. In this case, the cooling module 210 and the heat radiating fin 220 may not be provided.

Like the second embodiment, the second discharger 120C includes the second power supply 123. The second discharger 120C further includes a second discharge electrode 121C and a second counter electrode 122C. The second discharge electrode 121C is electrically connected to the second power supply 123. The second counter electrode 122C facing the second discharge electrode 121C is grounded.

The second discharger 120C includes a first water vessel 410 and a second water vessel 420. The second discharge electrode 121C includes a first proximal end 431, which is connected to the first water vessel 410, and a first distal end 432, which faces the second counter electrode 122C. The second counter electrode 122C includes a second proximal end 441, which is connected to the second water vessel 420, and a second distal end 442 facing the first distal end 432. The second discharge electrode 121C and the second counter electrode 122C are tubular. The second discharge electrode 121C and the second counter electrode 122C are exemplified as the tubular electrode.

Water is stored in the first water vessel 410. Due to a head pressure in the first water vessel 410, the water is pushed away from the first distal end 432 of the second discharge electrode 121C.

Water is stored in the second water vessel 420. Due to a head pressure in the second water vessel 420, the water is pushed away from the second distal end 442 of the second counter electrode 122C.

In the present embodiment, the first and second water vessels 410, 420 and the cooling module 210 are exemplified as the liquid feeder. The first and second water vessels 410, 420 are exemplified as the second feeder.

In the present embodiment, the first and second water vessels 410, 420 supply water to the second discharge electrode 121C and the second counter electrode 122C, respectively. Alternatively, another type of liquid may be supplied to the second discharge electrode 121C and the second counter electrode 122C.

In the present embodiment, the first and second water vessels 410, 420 supply water to the second discharge electrode 121C and the second counter electrode 122C, respectively. Alternatively, water may be supplied to one of the second discharge electrode 121C and the second counter electrode 122C.

Like the second embodiment, the second power supply 123 applies an AC voltage to the second discharge electrode 121C to cause a glow discharge between the first and second distal ends 432, 442. Since the glow discharge occurs near the upper end of the cylinder 119, fluid flowing into the cylinder 119 from the discharge space between the first discharge electrode 111 and the first counter electrode 112 is exposed to the glow discharge.

Since water flows from the first and second distal ends 432, 442 as described above, radicals generated by the glow discharge are included in particulate water. Therefore, not only the first discharger 110 but also the second discharger 120C may generate charged particulate water.

### <Fifth Embodiment>

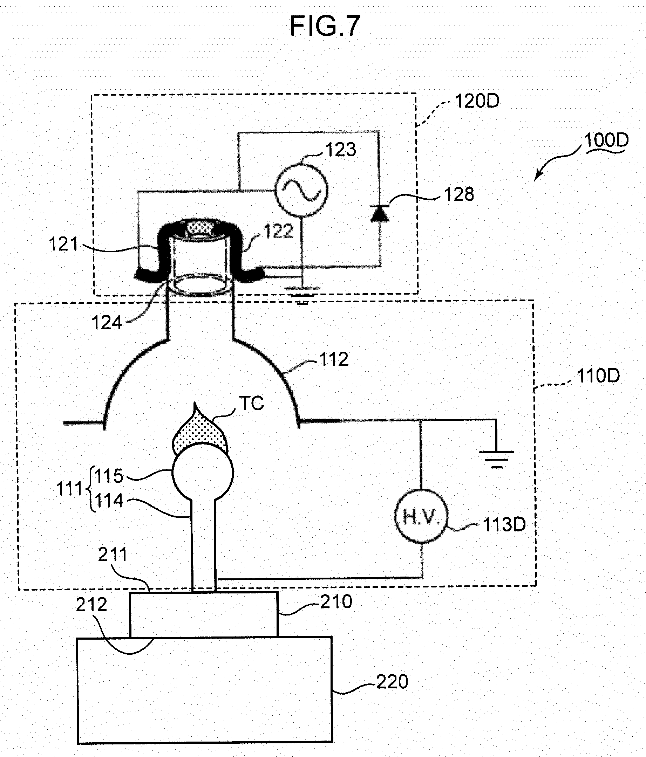

Fig. 7 is a schematic view of an electrostatic atomizer 100D exemplified as the active ingredient generator according to the fifth embodiment. The electrostatic atomizer 100D is described with reference to Fig. 7. The same components as the second embodiment are denoted by the same reference numerals. The description of the second embodiment is applicable to the components denoted by the same reference numerals as the second embodiment.

Like the second embodiment, the electrostatic atomizer 100D includes the cooling module 210 and the heat radiating fin 220. The electrostatic atomizer 100D further includes a first discharger 110D and a second discharger 120D.

Like the second embodiment, the first discharger 110D includes the first discharge electrode 111 and the first counter electrode 112. The first discharger 110D further includes a first power supply 113D. The body 114 of the first discharge electrode 111 is electrically connected to the first power supply 113D.

Unlike the second embodiment, the first power supply 113D applies a DC voltage to the first discharge electrode 111, the DC voltage being high enough to cause a discharge. Accordingly, a corona discharge happens to the head 115 connected to the upper end of the body 114.

Like the second embodiment, the second discharger 120D includes the second discharge electrode 121, the second counter electrode 122, the second power supply 123 and the supporting cylinder 124. The second discharger 120D further includes a diode 128 connected in parallel to the second power supply 123.

The diode 128 gives a bias to a voltage signal output from the second power supply 123. Therefore, the diode 128 may periodically generate a potential difference high enough to cause a glow discharge between the second discharge electrode 121 and the second counter electrode 122. This means that an excessively high output level is not required for the second power supply 123.

### <Sixth Embodiment>

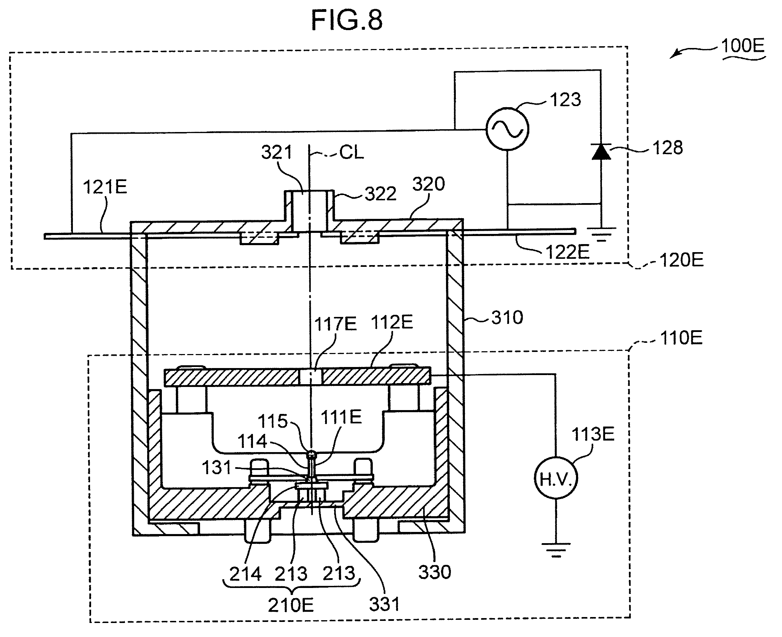

Fig. 8 is a schematic view of an electrostatic atomizer 100E exemplified as the active ingredient generator according to the sixth embodiment. The electrostatic atomizer 100E is described with reference to Fig. 8. The same components as the fifth embodiment are denoted by the same reference numerals. The description of the fifth embodiment is applicable to the components denoted by the same reference numerals as the fifth embodiment.

The electrostatic atomizer 100E includes a housing 310, a lid 320, a supporting structure 330, a first discharger 110E, a second discharger 120E and a cooling module 210E.

The first discharger 110E includes a first discharge electrode 111E, a first counter electrode 112E and a first power supply 113E. The first counter electrode 112E faces the first discharge electrode 111E. The first power supply 113E applies a DC voltage to the first counter electrode 112E, the DC voltage being high enough to cause a discharge. Accordingly, a corona discharge happens to the head 115 connected to the upper end of the body 114. The first discharge electrode 111E has a rod shape whereas the first counter electrode 112E has a planar shape. An opening 117E is formed in the first counter electrode 112E.

Like the fifth embodiment, the first discharge electrode 111E includes the body 114 and the head 115. The first discharge electrode 111E further includes a proximal end 131. The head 115 is closer to the first counter electrode 112E than the proximal end 131 is. Fig. 8 shows a center line CL which extends through the proximal end 131 and the head 115. The body 114 extends from the proximal end 131 to the head 115 along the center line CL. In the present embodiment, the head 115 is exemplified as the distal end. The center line CL is exemplified as the first line.

The supporting structure 330 supports the first discharge electrode 111E, the first counter electrode 112E and the cooling module 210E in the housing 310. The shape and structure of the supporting structure 330 do not limit principles of the present embodiment.

The opening 117E of the first counter electrode 112E supported by the supporting structure 330 is formed around the center line CL. Therefore, particulate water, radicals and ozone generated by a discharge between the first discharge electrode 111E and the first counter electrode 112E are emitted upward through the opening 117E.

The supporting structure 330 includes a heat radiating plate 331 situated under the first discharge electrode 111E. The cooling module 210E includes Peltier elements 213 and a cooling plate 214. The Peltier elements 213 are fixed onto the heat radiating plate 331. The cooling plate 214 is mounted on the Peltier elements 213. Therefore, the Peltier elements 213 are situated between the heat radiating plate 331 and the cooling plate 214. The proximal end 131 of the first discharge electrode 111E is fixed onto the cooling plate 214.

Like the fifth embodiment, the second discharger 120E includes the second power supply 123 and the diode 128. The second discharger 120E further includes a second discharge electrode 121 E and a second counter electrode 122E. The second counter electrode 122E faces the second discharge electrode 121 E. The second power supply 123 may cause a glow discharge between the second discharge electrode 121 E and the second counter electrode 122E.

As shown in Fig. 8, the center line CL extends between the second discharge electrode 121E and the second counter electrode 122E. Therefore, the glow discharge occurs around the center line CL.

The housing 310 is open upward. The lid 320 is attached to the upper edge of the housing 310. The lid 320 partially closes the housing 310. The second discharge electrode 121E and the second counter electrode 122E are attached to the upper edge of the housing 310. The second discharge electrode 121E and the second counter electrode 122E extend along the lower surface of the lid 320 toward the center line CL. The lid 320 may hold the second discharge electrode 121E and the second counter electrode 122E in cooperation with the housing 310.

The lid 320 includes a cylindrical portion 322 which defines an opening 321 around the center line CL. Particulate water, radicals and ozone generated by a discharge between the first discharge electrode 111E and the first counter electrode 112E are emitted upward through the opening 117E. The particulate water, radicals and ozone are then exposed to a glow discharge between the second discharge electrode 121 E and the second counter electrode 122E. The glow discharge reduces ozone whereas the glow discharge increases radicals. The particulate water, the increased radicals and the reduced ozone are discharged outside the housing 310 after the exposure to the glow discharge.

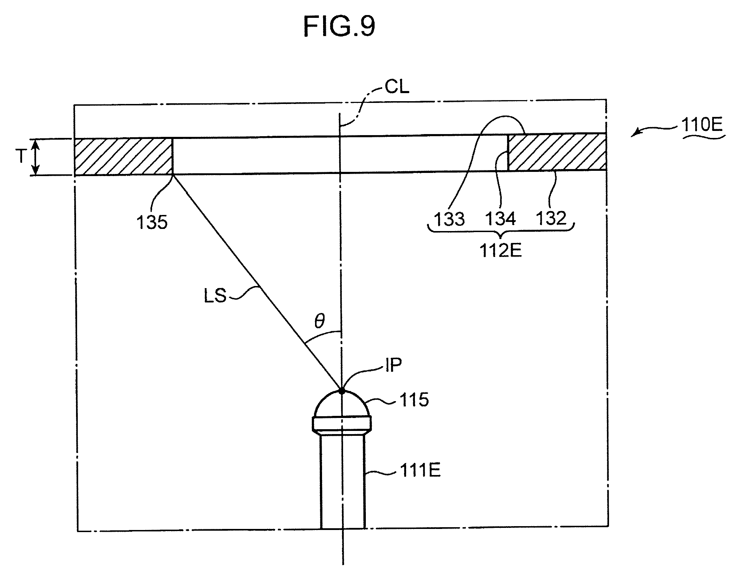

Fig. 9 is a partial enlarged view of the first discharger 110E around the center line CL. The first discharger 110E is described with reference to Fig. 9.

The first counter electrode 112E includes a lower surface 132, which faces the first discharge electrode 111E, an upper surface 133 opposite to the lower surface 132, and an edge surface 134, which defines a contour of the opening 117E. The edge surface 134 and the lower surface 132 define a lower corner edge 135. In the present embodiment, the lower corner edge 135 is exemplified as the edge.

Fig. 9 shows an intersection IP of the center line CL with the head 115. Fig. 9 further shows a line LS connecting the intersection IP to the lower corner edge 135 and an angle θ (θ≤90°) formed between the line LS and the center line CL. The angle θ affects concentration of an electric field generated between the first discharge electrode 111 E and the first counter electrode 112E. If the angle θ is large, there is little concentration of an electric field between the first discharge electrode 111E and the first counter electrode 112E. If the angle θ is small, there is a concentrated electric field between the first discharge electrode 111E and the first counter electrode 112E. The concentrated electric field results in a high velocity of ion flow generated by a discharge which occurs between the first discharge electrode 111E and the first counter electrode 112E. In the present embodiment, the line LS is exemplified as the second line.

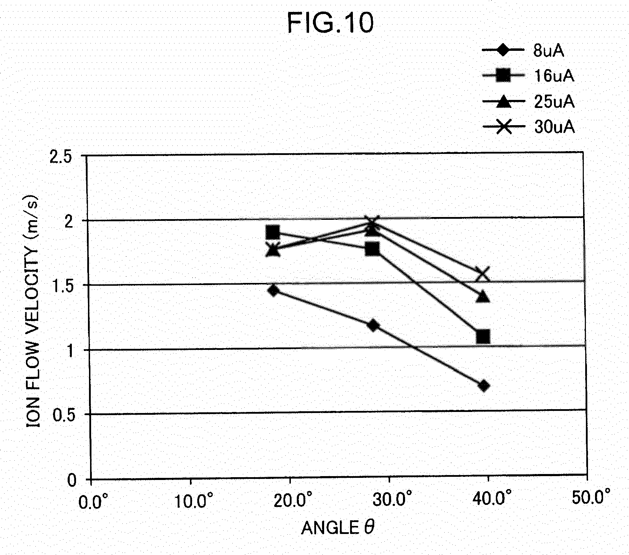

Fig. 10 is a schematic graph showing a relationship between the angle θ and a velocity of ion flow. The relationship between the angle θ and the velocity of ion flow is described with reference to Figs. 9 and 10.

The present inventors made each of three planar electrodes different in opening diameter face the first discharge electrode, and set conditions 20°, 30° and 40° for the angle 9. A velocity of ion flow caused by a discharge was then measured. The present inventors set four conditions (8 µA, 16 µA, 25 µA, and 30 µA) for a magnitude of current flowing between the electrodes.

A high velocity of ion flow was measured under the condition in which the angle θ was 20°. A higher velocity of ion flow was measured under the condition of "angle θ=30°" and "inter-electrode current≥25 µA " than the condition of "angle θ=20°". A lower velocity of ion flow was measured under the condition of "angle θ=30°" and "inter-electrode current<25 µA " than the condition of "angle θ=20°". A lower velocity of ion flow was measured under the condition in which the angle θ was 40° than the condition of "angle θ=20°". Therefore, it may be figured out that the angle θ of 30° or smaller is preferable for obtaining a high velocity of ion flow.

If the angle θ is set appropriately, there is a high velocity of ion flow. Therefore, the blower described in the context of the third embodiment may not be required.

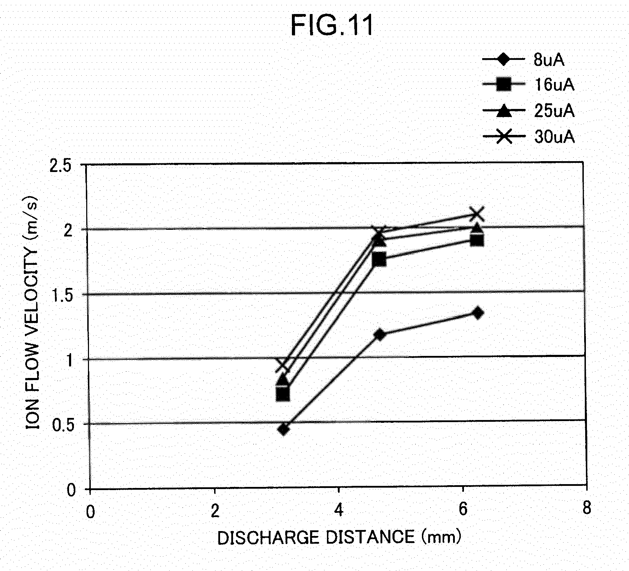

Fig. 11 is a schematic graph showing a relationship between a discharge distance and an ion flow velocity. The relationship between the discharge distance and the ion flow velocity is described with reference to Figs. 9 and 11.

The term "discharge distance" shown in Fig. 11 corresponds to a length of the line LS described with reference to Fig. 9. Ions generated by a discharge stay in an electric field for a long period if the discharge distance is large. Meanwhile, there is an increased velocity of ion flow since the ions are accelerated.

The present inventors set conditions of 2.5 mm, 4.5 mm and 6 mm for the discharge distance and measured an ion flow velocity under each of the conditions. As clearly shown by Fig. 11, a high velocity of ion flow is measured under the condition of "discharge distance≥4.5 mm" whereas it is observed that the velocity of ion flow decreases abruptly under the condition of "discharge distance<4.5 mm". Therefore, it may be figured out that a discharge distance of 4.5 mm or more is preferable for obtaining a high velocity of ion flow.

If the discharge distance is set appropriately, there is a high velocity of ion flow. Therefore, the blower described in the context of the third embodiment may not be required.

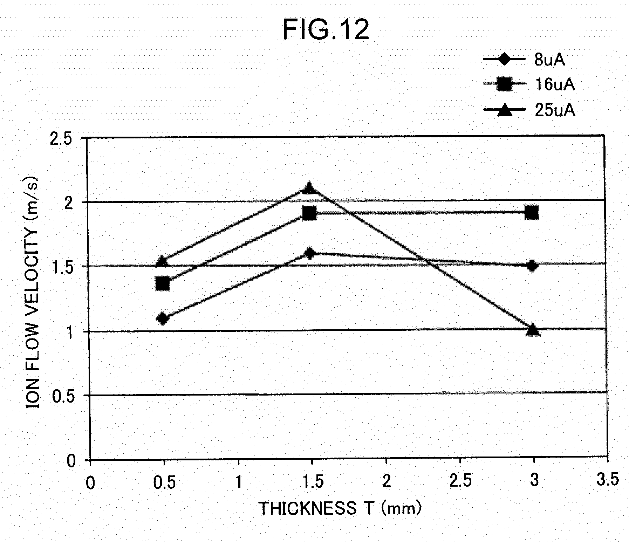

Fig. 12 is a schematic graph showing a relationship between a thickness of the first counter electrode 112E and an ion flow velocity. The relationship between a thickness of the first counter electrode 112E and the ion flow velocity is described with reference to Figs. 8 to 12.

In Fig. 9, a thickness (a dimension along the center line CL) of the first counter electrode 112E is represented by the symbol "T". When the thickness T of the first counter electrode 112E is large, there is an intensified electric field between the first discharge electrode 111E and the first counter electrode 112E. Accordingly, the ion flow velocity increases. However, if the thickness T of the first counter electrode 112E is too large, the first counter electrode 112E attracts a large amount of ions. Accordingly, an ion flow velocity decreases.

The present inventors prepared three planar electrodes which were different in thickness. The present inventors caused a discharge between the first discharge electrode 111E and each of these planar electrodes, and then measured an ion flow velocity.

With reference to Fig. 12, it may be figured out that an ion flow velocity is larger under a condition of "T=1.5 mm" than a condition of "T=0.5 mm". The ion flow velocity is maintained at a high level under a condition of "T≥1.5 mm" and "inter-electrode current≤16 µA" whereas the ion flow velocity decreases remarkably under a condition of "T≥1.5 mm" and "inter-electrode current>16 µA ". Therefore, it is preferable to set the thickness T of the first counter electrode 112E to a value close to 1.5 mm. According to the present inventors, when the thickness T of the first counter electrode 112E is set to a range of 1 mm to 2 mm, a velocity high enough to deliver ion flow to the second discharger 120E is obtained without a blower.

It may be figured out from the graphs shown in Figs. 10 to 12 that a high velocity of ion flow is obtained if a high current value is generated between the first discharge electrode 111E and the first counter electrode 112E. According to the present inventors, when an average value of the current generated between the first discharge electrode 111E and the first counter electrode 112E is in a range of 10 µA to 25 µA, a corona discharge occurs appropriately between the first discharge electrode 111 E and the first counter electrode 112E. On the other hand, if the average value of the current generated between the first discharge electrode 111E and the first counter electrode 112E is higher than 25 µA, both of a corona discharge and an arc discharge occur. With reference to Figs. 10 and 11, a large difference in the ion flow velocity is not found when the condition of 25 µA is compared with the condition of 30 µA. Therefore, it may be figured out that the condition of 30 µA provides poorer energy efficiency than the condition of 25 µA does. Accordingly, it is preferable that the average value of the current generated between the first discharge electrode 111E and the first counter electrode 112E is set to a range of 10 µA to 25 µA.

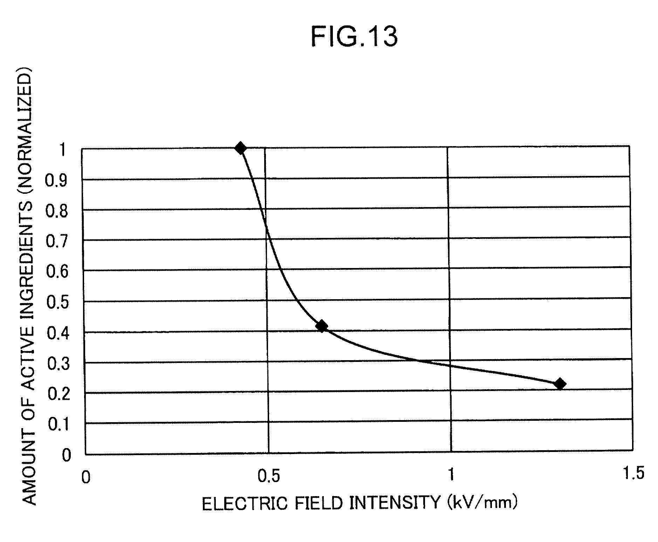

Fig. 13 is a schematic graph showing a relationship between electric field intensity, which appears between the first counter electrode 112E and a set of the second discharge electrode 121E and the second counter electrode 122E, and a yield of active ingredients. The relationship between the electric field intensity and the yield of active ingredients is described with reference to Figs. 8 and 13.

When a voltage is applied to the first and second dischargers 110E, 120E, an electric field is generated between the first counter electrode 112E and a set of the second discharge electrode 121E and the second counter electrode 122E. The electric field between the first counter electrode 112E and the set of the second discharge electrode 121E and the second counter electrode 122E may affect airflow which flows from the opening 117E formed in the first counter electrode 112E to the opening 321 of the cylindrical portion 322. As shown in Fig. 13, if the electric field between the first counter electrode 112E and the set of the second discharge electrode 121E and the second counter electrode 122E is excessively strong, a decreased amount of active ingredients may pass through the opening 321 of the cylindrical portion 322.

The vertical axis of the graph in Fig. 13 indicates an amount of active ingredients by the value of "1", the active ingredients passing through the opening 321 of the cylindrical portion 322 when the electric field between the first counter electrode 112E and the set of the second discharge electrode 121 E and the second counter electrode 122E is "approximately 0.4 kV/mm".

As shown in Fig. 13, if the electric field between the first counter electrode 112E and the set of the second discharge electrode 121E and the second counter electrode 122E is smaller than "approximately 0.6 kV/mm", a greatly increased amount of active ingredients may pass through the opening 321 of the cylindrical portion 322. Therefore, a voltage application to the first and second dischargers 110E, 120E may be controlled so that the electric field between the first counter electrode 112E and the set of the second discharge electrode 121E and the second counter electrode 122E is smaller than "approximately 0.6 kV/mm". If the electric field between the first counter electrode 112E and the set of the second discharge electrode 121E and the second counter electrode 122E is smaller than "approximately 0.5 kV/mm", 75% or more of active ingredients may pass through the opening 321 of the cylindrical portion 322. Therefore, a voltage application to the first and second dischargers 110E, 120E may be controlled so that the electric field between the first counter electrode 112E and the set of the second discharge electrode 121E and the second counter electrode 122E is smaller than "approximately 0.5 kV/mm".

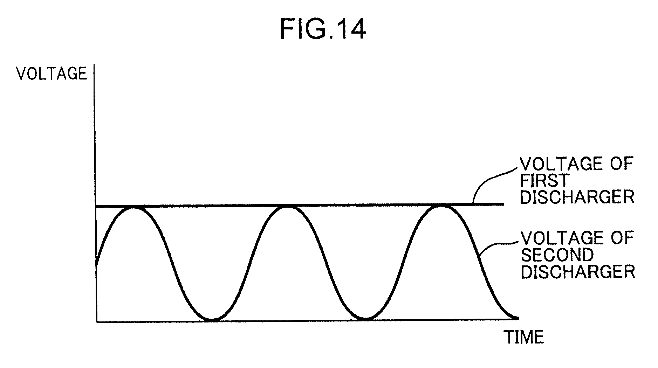

Fig. 14 is a graph showing an exemplary control of a voltage application to the first and second dischargers 110E, 120E. The control of a voltage application to the first and second dischargers 110E, 120E is described with reference to Figs. 8 and 14.

As shown in Fig. 14, if the second power supply 123 generates an AC voltage with a positive polarity, the first power supply 113E may generate a constant voltage with a positive polarity. Conversely, if the second power supply 123 generates an AC voltage with a negative polarity, the first power supply 113E may generate a constant voltage with a negative polarity. If the polarity of the voltage applied by the first power supply 113E is identical to the polarity of the voltage applied by the second power supply 123, the electric field between the first counter electrode 112E and the set of the second discharge electrode 121E and the second counter electrode 122E does not excessively increase. Therefore, there is an increased amount of active ingredients passing through the opening 321 of the cylindrical portion 322.

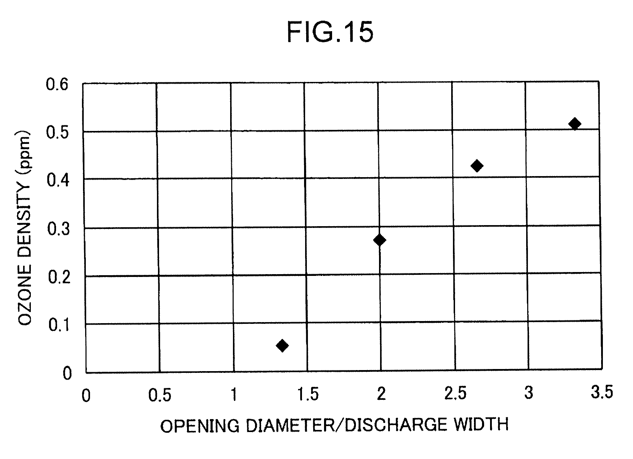

Fig. 15 is a schematic graph showing a relationship between a size of the opening 321 of the cylindrical portion 322 and a yield of ozone. The relationship between a size of the opening 321 of the cylindrical portion 322 and a yield of ozone is described with reference to Figs. 8 and 15.

As described above, a glow discharge between the second discharge electrode 121E and the second counter electrode 122E reduces an amount of ozone. As shown in Fig. 8, if there is the cylindrical portion 322 extending downstream in a flowing direction of the ion flow, the ozone reduction effect of the glow discharge also occurs in the cylindrical portion 322. If the cylindrical portion 322 is excessively large with respect to a distance between the second discharge electrode 121E and the second counter electrode 122E, there is an increased amount of ozone, which fails to be exposed to the glow discharge in a passage defined by the cylindrical portion 322. Therefore, a designer may set a size of the cylindrical portion 322 on the basis of a distance between the second discharge electrode 121E and the second counter electrode 122E.

The term "opening diameter" on the horizontal axis of the graph in Fig. 15 means a diameter of the opening 321 of the cylindrical portion 322. The term "discharge width" on the horizontal axis of the graph in Fig. 15 means a distance between the second discharge electrode 121E and the second counter electrode 122E. If the opening diameter is set to a value twice or less as long as the discharge width, ozone density is suppressed to be smaller than "0.3 ppm". Therefore, a designer may set the opening diameter to a value twice or less as long as the discharge width. The designer may set a lower limit of the opening diameter by taking account of a resistance of the cylindrical portion 322 to ion flow and other design conditions. For example, the designer may set the opening diameter to the same value as the discharge width.

### <Seventh Embodiment>

As described in the context of the sixth embodiment, the generation of strong ion flow contributes to emission of active ingredients from the housing. Techniques for intensifying the ion flow are described in the seventh embodiment.

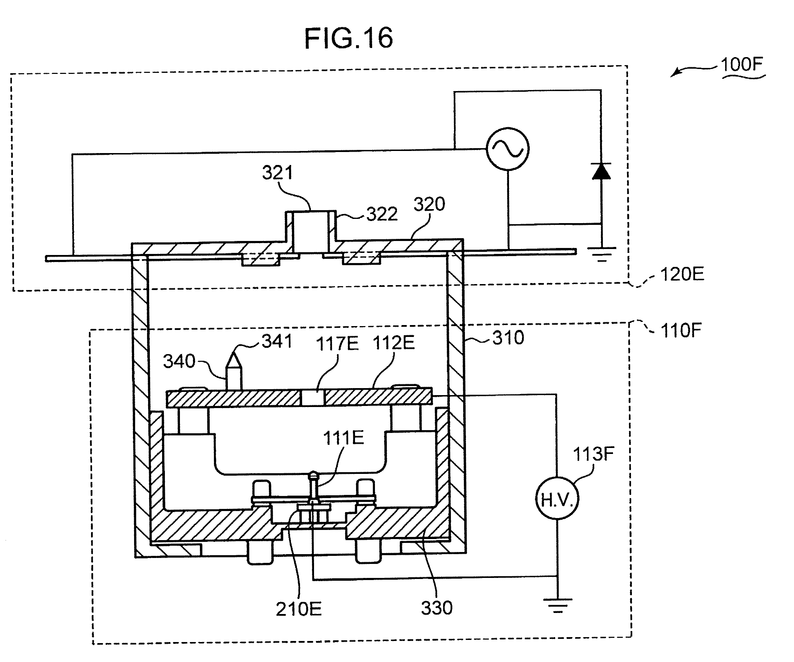

Fig. 16 is a schematic view of an electrostatic atomizer 100F exemplified as the active ingredient generator according to the seventh embodiment. The electrostatic atomizer 100F is described with reference to Fig. 16. The same components as the sixth embodiment are denoted by the same reference numerals. The description of the sixth embodiment is applicable to the components denoted by the same reference numerals as the sixth embodiment.

Like the sixth embodiment, the electrostatic atomizer 100F includes the housing 310, the lid 320, the supporting structure 330, the second discharger 120E and the cooling module 210E. The electrostatic atomizer 100F further includes a first discharger 110F.

Like the sixth embodiment, the first discharger 110F includes the first discharge electrode 111E and the first counter electrode 112E. The first discharger 110F further includes a first power supply 113F. The first power supply 113F sets a potential of the first discharge electrode 111E to a ground level and applies a high voltage to the first counter electrode 112E.

The first discharger 110F further includes a needle electrode 340 which extends upward from the first counter electrode 112E. The needle electrode 340 includes a distal end 341 which becomes sharp upward. When the first power supply 113F applies a high voltage to the first counter electrode 112E, a high voltage is also applied to the needle electrode 340 connected to the first counter electrode 112E. Accordingly, an electric field concentrates on the distal end 341 to cause a discharge from the distal end 341. The discharge from the distal end 341 results in an upward ion flow. Since the needle electrode 340 generates an additional ion flow, the electrostatic atomizer 100F may easily cause a high velocity of ion flow. In the present embodiment, the needle electrode 340 is exemplified as the ion flow generation electrode.

The ion flow generation electrode may not be a needle shape. A designer may give the ion flow generation electrode various shapes configured to cause ion flow. The electrostatic atomizer may include a plurality of ion flow generation electrodes. The principles of the present embodiment are not limited by how many ion flow generation electrodes are.

The ion flow generation electrode may be situated at a different position from the needle electrode 340. The ion flow generation electrode may be situated at an appropriate position for design of an electrostatic atomizer. Therefore, the ion flow generation electrode may cause a discharge under electrical energy supply from another power supply different from the first power supply 113F. If there is increased ion flow from the opening 117E of the first counter electrode 112E to the opening 321 of the cylindrical portion 322, the position of the ion flow generation electrode and a circuit structure for the electrical energy supply to the ion flow generation electrode do not limit the principles of the present embodiment.

### <Eighth Embodiment>

As described in the context of the sixth embodiment, the electric field generated between the first and second dischargers may interfere with emission of active ingredients. In order to weaken the electric field between the first and second dischargers, a designer may increase a distance between the first and second dischargers. However, such a design results in an increase in a size of the active ingredient generator. Techniques for making the electric field between the first and second dischargers less influential to emission of active ingredients are described in the eighth embodiment.

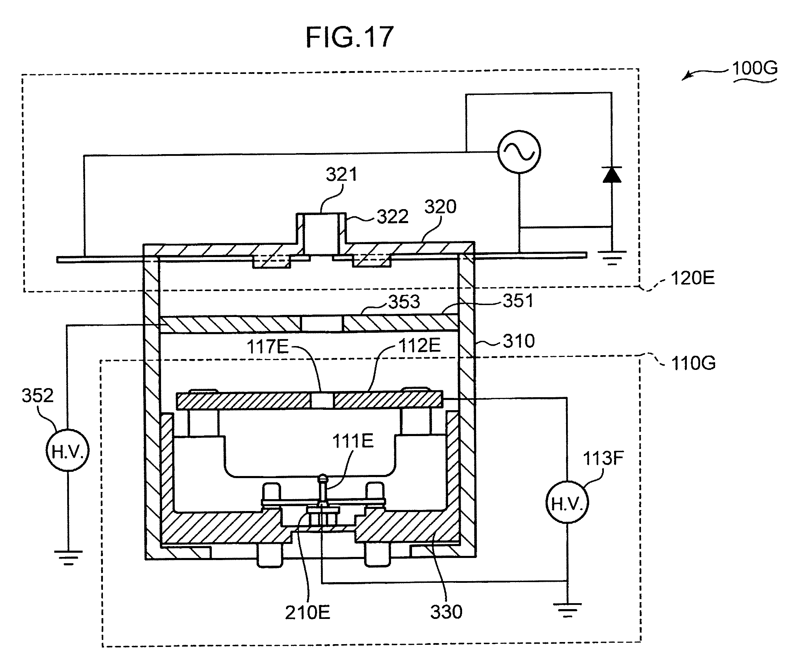

Fig. 17 is a schematic view of an electrostatic atomizer 100G exemplified as the active ingredient generator according to the eighth embodiment. The electrostatic atomizer 100G is described with reference to Fig. 17. In the eighth embodiment, the same components as the sixth to eighth embodiments are denoted by the same reference numerals. The description of the sixth or seventh embodiment is applicable to the components denoted by the same reference numerals.

Like the sixth embodiment, the electrostatic atomizer 100G includes the housing 310, the lid 320, the supporting structure 330, the second discharger 120E and the cooling module 210E. The electrostatic atomizer 100G further includes a first discharger 110G.

Like the seventh embodiment, the first discharger 110G includes the first discharge electrode 111E, the first counter electrode 112E and the first power supply 113F.

The electrostatic atomizer 100G further includes an intermediate electrode 351 and a third power supply 352. The intermediate electrode 351 is held between the first and second dischargers 110G, 120E by the housing 310. An opening 353 is formed in the intermediate electrode 351. The opening 353 is aligned vertically with the opening 117E of the first counter electrode 112E and the opening 321 of the cylindrical portion 322. Therefore, active ingredients pass through the openings 117E, 353, 321 sequentially, and are then emitted from the housing 310.

The third power supply 352 applies a high voltage (a voltage value between applied voltage values in the first and second dischargers 110G, 120E) to the intermediate electrode 351. Accordingly, the intermediate electrode 351 is maintained at a constant potential. Therefore, an electric field between the first and second dischargers 110G, 120E becomes less influential to flow of active ingredients passing the openings 117E, 353, 321 sequentially.

The intermediate electrode may be set to an open potential. In this case, the intermediate electrode may not be connected to a power supply device configured to apply a high voltage. Alternatively, the intermediate electrode may be connected to the ground. In this case, the intermediate electrode is set to a ground potential.

A member formed from a nonconductive material such as a resin may be used instead of the intermediate electrode. The nonconductive member reduces a passage amount of electric field between the first and second dischargers 110G, 120E. Accordingly, an excessively strong electric field is less likely to be generated between the first and second dischargers 110G, 120E. Therefore, active ingredients are stably emitted from the housing 310.

Various active ingredient generators may be designed and manufactured on the basis of the principles of the aforementioned various embodiments. Various changes, improvements or omissions may be made for performance required for active ingredient generators and applications of active ingredient generators. For example, a designer may deploy a plurality of discharge electrodes in the first discharger. In this case, a high-velocity ion flow may be generated.

The aforementioned embodiments mainly include the following features.

An active ingredient generator according to one aspect of the aforementioned embodiments includes a first discharger defining a discharge space in which a first discharge is caused, the first discharge having first energy; and a second discharger configured to cause a second discharge in a passage space which allows passage of fluid flowing from the discharge space, the second discharge having second energy larger than the first energy.

According to the aforementioned configuration, the first discharger causes the first discharge having the first energy. Accordingly, radicals and ozone are generated in the discharge space. The fluid flowing from the discharge space passes through the passage space. Meanwhile, the second discharger causes the second discharge having the second energy larger than the first energy. Consequently, ozone is reduced.

In the aforementioned configuration, the second discharge may be a glow discharge.

According to the aforementioned configuration, since the second discharger causes a glow discharge, ozone is reduced efficiently.

In the aforementioned configuration, the active ingredient generator may further include a liquid feeder configured to feed liquid to at least one of the first and second dischargers.

According to the aforementioned configuration, since the liquid feeder feeds liquid to at least one of the first and second dischargers, there is generation of charged particulate liquid containing radicals. Since the liquid surrounding the radicals protects the radicals from the second discharge, an amount of radicals delivered from the discharge space is less likely to decrease.

In the aforementioned configuration, the first discharger may include a first discharge electrode and a first counter electrode which faces the first discharge electrode. The first discharge may occur between the first discharge electrode and the first counter electrode. The liquid feeder may include a first feeder which feeds the liquid to the first discharge electrode.

According to the aforementioned configuration, since the first feeder feeds liquid to the first discharger, charged particulate liquid containing radicals is generated by the first discharge. Since the liquid surrounding the radicals protects the radicals from the second discharge, an amount of radicals delivered from the discharge space is less likely to decrease.

In the aforementioned configuration, the second discharger may include a second discharge electrode and a second counter electrode which faces the second discharge electrode. The fluid may be exposed to the second discharge caused between the second discharge electrode and the second counter electrode.

According to the aforementioned configuration, since gas flowing from the discharge space into the passage space is exposed to the second discharge caused between the second discharge electrode and the second counter electrode, ozone is reduced efficiently.

In the aforementioned configuration, the liquid feeder may include a second feeder which feeds the liquid to at least one electrode of the second discharge electrode and the second counter electrode.

According to the aforementioned configuration, since the second feeder feeds liquid to at least one electrode of the second discharge electrode and the second counter electrode, charged particulate liquid containing radicals is generated by the second discharge.

In the aforementioned configuration, the at least one electrode may include a tubular electrode. The second feeder may cause the liquid to flow into the tubular electrode.

According to the aforementioned configuration, since the second feeder feeds liquid into the tubular electrode, charged particulate liquid containing radicals is generated by the second discharge.

In the aforementioned configuration, the active ingredient generator may further include a blowing portion which causes airflow to make the fluid flow from the discharge space to the passage space.

According to the aforementioned configuration, since the blowing portion causes the airflow to make the fluid flow from the discharge space to the passage space, the fluid is exposed to the second discharge efficiently.

In the aforementioned configuration, the active ingredient generator may further include a blowing portion which causes airflow to make the fluid flow from the discharge space to the passage space. The first feeder may include a cooler, which causes condensation on the first discharge electrode, and a radiator, which causes heat radiation from the cooler. The radiator may be exposed to the airflow.

According to the aforementioned configuration, since the blowing portion causes the airflow to make the fluid flow from the discharge space to the passage space, the fluid is exposed to the second discharge efficiently.

Since condensation on the first discharge electrode is caused by the cooler, charged particulate water containing radicals is generated by the first discharge. Since water covering the radicals protects the radicals from the second discharge, an amount of radicals delivered from the discharge space to the passage space is less likely to decrease.

The radiator causes radiation of heat, which is generated in the cooler to cool the first discharge electrode. Since the radiator is exposed to the airflow generated by the blowing portion, there is very efficient heat dissipation.

In the aforementioned configuration, the first discharger may be a combination of a first discharge electrode and a first counter electrode which faces the first discharge electrode. The first counter electrode may be a planar electrode having an opening.

According to the aforementioned configuration, ion flow is caused by the first discharge. Since the counter electrode is a planar electrode having an opening, a velocity of ion flow flowing from the discharge electrode to the counter electrode increases even under the absence of the blowing portion. Therefore, the ion flow is efficiently exposed to the second discharge caused by the second discharger.

In the aforementioned configuration, the first discharge electrode may include a proximal end and a distal end which is closer to the first counter electrode than the proximal end is. The first counter electrode may include an edge which defines a contour of the opening. An angle between a first line extending from the proximal end to the distal end and a second line extending from the intersection of the first line with the distal end to the edge may be no more than 30°.

According to the aforementioned configuration, since the angle between the first line extending from the proximal end to the distal end and the second line extending from the intersection of the first line with the distal end to the edge is set to no more than 30°, there ion flow at high speed.

In the aforementioned configuration, a distance between the edge and the intersection may be set to a value of 4.5 mm or more.

According to the aforementioned configuration, since a distance between the edge and the intersection is set to a value of 4.5 mm or more, the ion flow is accelerated appropriately.

In the aforementioned configuration, the first counter electrode may be set to a range of 1 mm to 2 mm in thickness.

According to the aforementioned configuration, since the first counter electrode is set to a range of 1 mm to 2 mm in thickness, there is ion flow at high speed.

In the aforementioned configuration, an average value of current flowing between the first discharge electrode and the first counter electrode during the first discharge may be set to a range of 10 µA to 25 µA.

According to the aforementioned configuration, since the average value of current flowing between the first discharge electrode and the first counter electrode during the first discharge is set to a range of 10 µA to 25 µA, active ingredients are generated efficiently under a stable corona discharge.

In the aforementioned configuration, the first discharger may include a plurality of discharge electrodes.

According to the aforementioned configuration, since the first discharger includes a plurality of discharge electrodes, there is ion flow at high speed.

In the aforementioned configuration, the first discharge may cause ion flow from the opening to the second discharger. The first discharger may include at least one ion flow generation electrode to intensify the ion flow.

According to the aforementioned configuration, since the first discharger includes at least one ion flow generation electrode to intensify the ion flow, there is ion flow at high speed.

In the aforementioned configuration, the ion flow generation electrode may protrude from the first counter electrode toward the second discharger.

According to the aforementioned configuration, since the ion flow generation electrode protrudes from the first counter electrode toward the second discharger, the ion flow flowing from the opening toward the second discharger is accelerated efficiently.

In the aforementioned configuration, an electric field generated between the first and second dischargers may be set to no more than 0.6 kV/mm.

According to the aforementioned configuration, since the electric field generated between the first and second dischargers is set to no more than 0.6 kV/mm, the electric field generated between the first and second dischargers becomes less influential to emission of active ingredients.

In the aforementioned configuration, the active ingredient generator may further include an intermediate electrode situated between the first and second dischargers. The intermediate electrode may be maintained at a predetermined potential.

According to the aforementioned configuration, since the intermediate electrode is maintained at a predetermined potential, the electric field generated between the first and second dischargers becomes less influential to emission of active ingredients.

In the aforementioned configuration, the active ingredient generator may further include a nonconductive member situated between the first and second dischargers.

According to the aforementioned configuration, since there is the nonconductive member between the first and second dischargers, the electric field generated between the first and second dischargers becomes less influential to emission of active ingredients.

In the aforementioned configuration, the first and second discharges may be caused under voltage applications which are equal to each other in polarity.

According to the aforementioned configuration, an excessively strong electric field is not generated between the first and second dischargers. Therefore, the electric field generated between the first and second dischargers becomes less influential to emission of active ingredients.

In the aforementioned configuration, the active ingredient generator may further include a cylindrical portion defining a passage which allows passage of the ion flow exposed to the second discharge.

According to the aforementioned configuration, ozone and electrons resulting from the second discharge are likely to collide in the passage.

In the aforementioned configuration, the second discharger may include a second discharge electrode and a second counter electrode which faces the second discharge electrode. An opening diameter of the cylindrical portion may be set to be twice or less as long as a distance between the second discharge electrode and the second counter electrode.

According to the aforementioned configuration, since the opening diameter of the cylindrical portion is set to be twice or less as long as a distance between the second discharge electrode and the second counter electrode, ozone and electrons resulting from the second discharge are likely to collide in the passage.

### Industrial Applicability

The principles of the aforementioned embodiments are preferably used in a device which uses radicals to cause useful effects such as deodorization and sterilization.

## Claims

1. An active ingredient generator (100-100G) comprising:
a first discharger (110, 110D-110G) defining a discharge space in which a first discharge is caused, the first discharge having first energy; and
a second discharger (120, 120C-120E) configured to cause a second discharge in a passage space which allows passage of fluid flowing from the discharge space, the second discharge having second energy larger than the first energy,
**characterized by**
a liquid feeder (210, 210E, 410, 420) configured to feed liquid to at least one of the first and second dischargers (110, 110D-110G; 120, 120C-120E),
wherein the second discharge is a glow discharge.

2. The active ingredient generator according to claim 1,
wherein the first discharger (110, 110D-110G) includes a first discharge electrode (111, 111 E) and a first counter electrode (112, 112E) which faces the first discharge electrode (111, 111E),
wherein the first discharge occurs between the first discharge electrode (111, 111 E) and the first counter electrode (112, 112E), and
wherein the liquid feeder (210, 210E, 410, 420) includes a first feeder (210, 210E) which feeds the liquid to the first discharge electrode (111, 111 E).

3. The active ingredient generator according to claim 2,
wherein the second discharger (120, 120C-120E) includes a second discharge electrode (121, 121C, 121E) and a second counter electrode (122, 122C, 122E) which faces the second discharge electrode (121, 121C, 121E), and
wherein the fluid is exposed to the second discharge caused between the second discharge electrode (121, 121C, 121 E) and the second counter electrode (122, 122C, 122E).

4. The active ingredient generator according to claim 3,
wherein the liquid feeder (210, 210E, 410, 420) includes a second feeder (410, 420) which feeds the liquid to at least one electrode of the second discharge electrode (121, 121C, 121 E) and the second counter electrode (122, 122C, 122E).

5. The active ingredient generator according to claim 4,
wherein the at least one electrode includes a tubular electrode, and
wherein the second feeder (410, 420) causes the liquid to flow into the tubular electrode.

6. The active ingredient generator according to any one of claims 1 to 5, further comprising:
a blowing portion (300) which causes airflow to make the fluid flow from the discharge space to the passage space.

7. The active ingredient generator according to claim 2 or 3, further comprising:
a blowing portion (300) which causes airflow to make the fluid flow from the discharge space to the passage space,
wherein the first feeder (210, 210E) includes a cooler (210, 210E), which causes condensation on the first discharge electrode (111, 111 E), and a radiator (331), which causes heat radiation from the cooler (210, 210E), and
wherein the radiator (331) is exposed to the airflow.

## Patentansprüche

1. Vorrichtung (100-100G) zum Erzeugen aktiver Substanzen, die umfasst:
eine erste Entladungseinrichtung (110, 110D-110G), die einen ersten Entladungsraum bildet, in dem eine erste Entladung veranlasst wird, wobei die erste Entladung erste Energie hat; und
eine zweite Entladungseinrichtung (120, 120C-120E), die so ausgeführt ist, dass sie eine zweite Entladung in einem Durchlassraum veranlasst, der Fluid durchlässt, das aus dem Entladungsraum strömt, wobei die zweite Entladung zweite Energie hat, die größer ist als die erste Energie,
**gekennzeichnet durch**
eine Einrichtung (210, 210E, 410, 420) zum Zuführen von Flüssigkeit, die so ausgeführt ist, dass sie der ersten oder/und der zweiten Entladungseinrichtung (110, 110D-110G; 120, 120C-120E) Flüssigkeit zuführt,
wobei die zweite Entladung eine Glimmentladung ist.

2. Vorrichtung zum Erzeugen aktiver Substanzen nach Anspruch 1,
wobei die erste Entladungseinrichtung (110, 110D-110G) eine erste Entladungselektrode (111, 111 E) sowie eine erste Gegenelektrode (112, 112E) einschließt, die der ersten Entladungselektrode (111, 111 E) zugewandt ist,
und die erste Entladung zwischen der ersten Entladungselektrode (111, 111 E) und der ersten gegen Elektrode (112, 112E) stattfindet, und
die Einrichtung (210, 210E, 410, 420) zum Zuführen von Flüssigkeit eine erste Zuführeinrichtung (210, 210E) einschließt, die die Flüssigkeit der ersten Entladungselektrode (111, 111 E) zuführt.

3. Vorrichtung zum Erzeugen aktiver Substanzen nach Anspruch 2,
wobei die zweite Entladungseinrichtung (120, 120C-120E) eine zweite Entladungselektrode (121, 121C, 121E) sowie eine zweite Gegenelektrode (122, 122C, 122E) einschließt, die der zweiten Entladungselektrode (121, 121C, 121 E) zugewandt ist, und wobei das Fluid der zweiten Entladung ausgesetzt ist, die zwischen der zweiten Entladungselektrode (121, 121C, 121 E) und der zweiten Gegenelektrode (122, 122C, 122E) veranlasst wird.

4. Vorrichtung zum Erzeugen aktiver Substanzen nach Anspruch 3,
wobei die Einrichtung (210, 210E, 410, 420) zum Zuführen von Flüssigkeit eine zweite Zuführeinrichtung (410, 420) einschließt, die die Flüssigkeit wenigstens einer Elektrode von der zweiten Entladungselektrode (121, 121C, 121 E) und der zweiten Gegenelektrode (122, 122C, 122E) zuführt.

5. Vorrichtung zum Erzeugen aktiver Substanzen nach Anspruch 4,
wobei die wenigstens eine Elektrode eine röhrenförmige Elektrode einschließt, und
die zweite Zuführeinrichtung (410, 420) bewirkt, dass die Flüssigkeit in die röhrenförmige Elektrode hineinströmt.

6. Vorrichtung zum Erzeugen aktiver Substanzen nach einem der Ansprüche 1 bis 5, die des Weiteren umfasst:
einen Blasabschnitt (300), der bewirkt, dass Luftstrom das Fluid aus dem Entladungsraum zu dem Durchlassraum strömen lässt.

7. Vorrichtung zum Erzeugen aktiver Substanzen nach Anspruch 2 oder 3, die des Weiteren umfasst:
einen Blasabschnitt (300), der bewirkt, dass Luftstrom das Fluid aus dem Entladungsraum zu dem Durchlassraum strömen lässt,
wobei die erste Zuführeinrichtung (210, 210E) einen Kühler (210, 210E), der Kondensation an der ersten Entladungselektrode (111, 111 E) bewirkt, sowie einen Strahler (331) einschließt, der Wärmestrahlung von dem Kühler (210, 210E) bewirkt, und
der Strahler (331) dem Luftstrom ausgesetzt ist.

## Revendications

1. Générateur d'ingrédient actif (100-100G) comprenant :
un premier dispositif de décharge (110, 110D-110G) définissant un espace de décharge dans lequel une première décharge est provoquée, la première décharge présentant une première énergie ; et
un second dispositif de décharge (120, 120C-120E) configuré pour entraîner une seconde décharge dans un espace de passage qui permet le passage de fluide s'écoulant depuis l'espace de décharge, la seconde décharge ayant une seconde énergie supérieure à la première énergie,
**caractérisé en ce que**
un dispositif d'alimentation en liquide (210, 210E, 410, 420) configuré pour alimenter un liquide au niveau d'au moins l'un des premiers et des seconds dispositifs de décharge (110, 110D-110G ; 120, 120C-120E),
où la seconde décharge est une décharge luminescente.

2. Générateur d'ingrédient actif selon la revendication 1,
où la première décharge (110, 110D-110G) inclut une première électrode de décharge (111, 111E) et une première électrode de référence (112, 112E) qui fait face à la première électrode de décharge (111, 111E),
où la première décharge se produit entre la première électrode de décharge (111, 111E) et la première électrode de référence (112, 112E), et
où le dispositif d'alimentation de liquide (210, 210E, 410, 420) inclut un premier dispositif d'alimentation (210, 210E) qui alimente le liquide au niveau de la première électrode de décharge (111, 111E).

3. Générateur d'ingrédient actif selon la revendication 2,
où le second dispositif de décharge (120, 120C-120E) inclut une seconde électrode de décharge (121, 121C, 121E) et une seconde électrode de référence (122, 122C, 122E) qui fait face à la seconde électrode de décharge (121, 121C, 121E), et
où le fluide est exposé à la seconde décharge provoquée entre la seconde électrode de décharge (121, 121C, 121E) et la seconde électrode de référence (122, 122C, 122E).

4. Générateur d'ingrédient actif selon la revendication 3,
où le dispositif d'alimentation de liquide (210, 210E, 410, 420) inclut un second dispositif d'alimentation (410, 420) qui alimente ledit fluide au niveau d'au moins une électrode de la seconde électrode de décharge (121, 121C, 121E) et de la seconde électrode de référence (122, 122C, 122E).

5. Générateur d'ingrédient actif selon la revendication 4,
où la au moins une électrode inclut une électrode tubulaire, et
le second dispositif d'alimentation (410, 420) entraîne l'écoulement du liquide dans l'électrode tubulaire.

6. Générateur d'ingrédient actif selon l'une quelconque des revendications 1 à 5, comprenant en outre :
une partie d'insufflation (300) qui entraîne un écoulement d'air faisant s'écouler le fluide depuis l'espace de décharge vers l'espace de passage.

7. Générateur d'ingrédient actif selon la revendication 2 ou 3, comprenant en outre :
une partie d'insufflation (300) qui entraîne un écoulement d'air faisant s'écouler le fluide depuis l'espace de décharge vers l'espace de passage,
où le premier dispositif d'alimentation (210, 210E) inclut un dispositif réfrigérant (210, 210E), qui provoque la condensation sur la première électrode de décharge (111, 111E), et un radiateur (331), qui provoque l'irradiation de chaleur depuis le dispositif réfrigérant (210, 210E), et
où le radiateur (331) est exposé à l'écoulement d'air.
